# EUROPEAN PATENT APPLICATION

(11) **EP 0 947 717 A2**
(43) Date of publication of application: **06.10.1999**
(21) Application number: 98830189.1
(22) Date of filing: 30.03.1998
(51) Int. Cl.: F16C 13/00, B21B 31/10

(54) **A processing unit for advancing articles**

(71) Applicant: Fameccanica. Data S.p.A., 65127 Pescara (IT)
(72) Inventor: De Angelis, Tonio, 65123 Pescara (IT)
(74) Representative: Bosotti, Luciano

(57) **Abstract**

A unit (10) comprising two motionally cooperating elements consisting, for example, of a roller (5)/counter-roller (3) pair. The roller (5) is mounted in accordance with a general cantilever configuration on the distal end (4b) of a respective shaft (4). Preferably, the said end (4b) is configured, together with a complementary part (5c) of the roller (5), in such a way as to realize a rapid fastening device. Preferably also, the counter-roller (3) is mounted on a mobile structure (8, 9) that will render it floating and thus enable it to adapt itself to possible flexures of the shaft (4) on which the roller (5) is mounted. The preferential application of the invention is to plants for the fabrication of such hygienic and sanitary products as sanitary towels and baby napkins.

## Description

The present invention relates to a processing unit comprising motionally cooperating elements in accordance with the preamble of Claim 1 hereinbelow.

Processing units of the type just described normally find application in industrial plants, especially plants for the fabrication of such hygienic and sanitary products as, for example, sanitary towels, diapers, diaper-panties and similar products. In the configuration that is currently in particularly common use, these units comprise a pair of elements mounted in accordance with a roller and counter-roller configuration on a structure that can be likened to a calander or mangle structure. The flow of products to be processed passes through the space defined by the two cooperating elements, one of which realizes the processing action in the proper sense of the term (i.e. cutting, shaping, welding, etc.).

Especially in plants operating at high product flow rates (as is typically the case of plants for the fabrication of hygienic and sanitary products) the need for arranging for the substitution of at least one of the cooperating elements arises with a certain frequency; as a general rule, it is the active element that has to be replaced, since it is more prone to suffering wear and tear due to use and/or will have to be substituted more frequently as a part and parcel of a "format change" operation.

Whenever such a change has to be effected in one of the traditional mounting solutions, it is necessary to dismantle at a part of the supporting structure, remove the element or elements that are to be replaced, place and fix into position the replacement element or elements and then - before resuming the production process - undertake all the alignment and adjustment operations required by the machine as a whole. In this connection it should also be borne in mind that the element or elements that have to be replaced are often associated with appropriate temperature control systems (heating/cooling) complete with all the necessary cables and/or piping. The substitution of the element or elements therefore implies that these cables and or pipes have to be disconnected and subsequently replaced as an auxiliary operation.

No matter in how efficient a manner these operations may be carried out, the necessary input of work and - above all - time is in no manner or wise compatible with the need for making the fullest possible use of the plant and the need for avoiding interruptions of the production processes for significant periods of time. For this reason some industrial establishments adopt the solution of providing the processing unit with a stand-by twin that will be activated as and when the principal unit has to be shut down for making such a substitution and thus avoiding an interruption of the production cycle. Apart from being rather costly to realize and in any case proving to be not particularly efficient in actual practice, a solution of this type does not usually provide a complete solution of the problems bound up with the presence of cables and/or pipes.

The present invention sets out to furnish a processing unit in which the aforesaid difficulties and drawbacks are overcome.

According to the present invention, this object is attained thanks to a processing unit having the characteristics specifically set out in the claims attached hereto.

The invention will now be described - though only by way of an example that is not in any way to be regarded as limitative - by reference to the attached drawings, where:

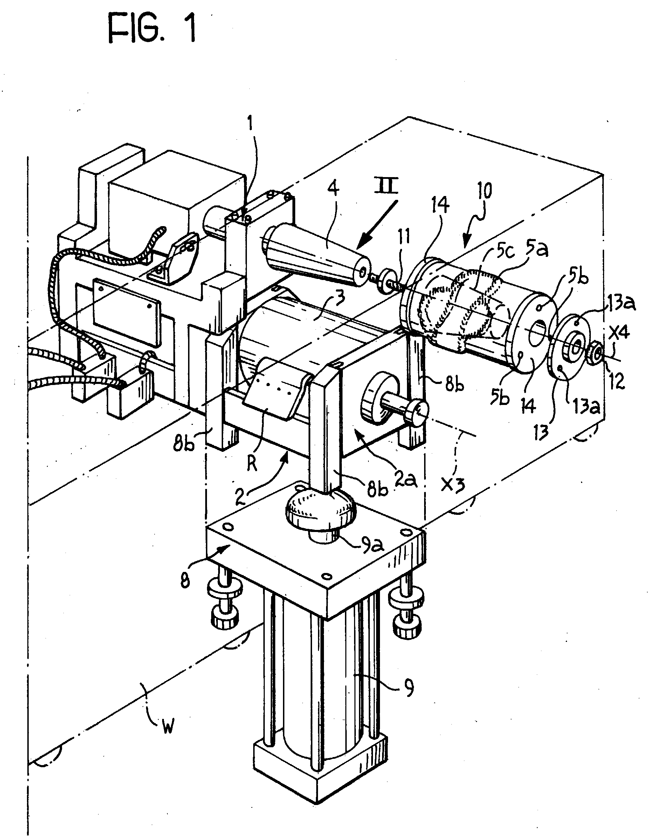

- Figure 1 shows a partially exploded perspective view of a processing unit in accordance with the invention, and
- Figure 2 shows a partially sectioned view to a larger scale of the part indicated by the arrow II in Figure 1.

In the attached drawings the reference number 10 indicates the whole of a processing unit constituted by - for example - an item of equipment comprised in a plant for the fabrication of hygienic and sanitary products and intended to realize on a continuous or substantially continuous strip or web of products (which as a general rule are still attached to each other in the worm of a strip or web W) an operation of welding and/or cutting that is intended to complete the formation of the individual products. In the specific case here illustrated, the unit in question could form part, for example, of a plant for the fabrication of sanitary towels of the type provided with lateral "wings".

In essential terms, the processing unit 10 is made up of a structure 1 , which is fixed, and a structure 2 that is at least partially mobile.

The structure 2 comprises a C-shaped frame or scaffolding of which the two lateral pieces are both provided with support bearings that sustain a roller 3 mounted in such a manner as to enable it to rotate about a horizontal axis X3. The roller (counter roller) may either be mounted as an idle roller or can be subjected to the action of a motor unit that is not specifically indicated on the drawings.

The reference number 4 indicates a shaft mounted on the fixed structure 1 that can rotate about a respective axis X4, which is likewise horizontal. In the example here illustrated, the axis X4 is arranged in a position exactly above the axis X3. Though this configuration is possible, it is not however in any way imperative.

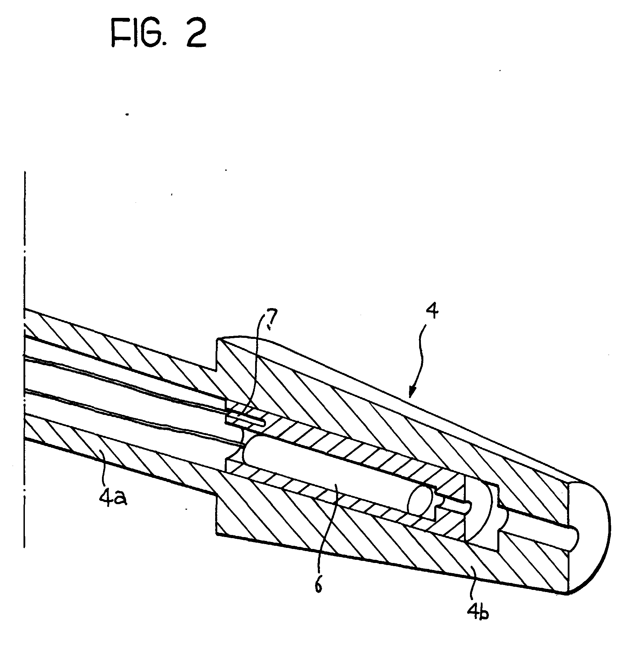

As can readily be seen in the enlarged detail of Figure 2, the shaft 4 comprises a root or proximal part 4a mounted and supported (in a known manner) within the fixed framework 1. The shaft 4 also comprises and external or distal part 4b that projects in the manner of a cantilever above the mobile structure 2 and is therefore located above the counter roller 3.

The distal part 4b of the shaft 4 is usually of a tapered shape and preferably has the conformation of a coupling or fastening (for example, under the form of an attachment of the type known as a cone fastening, for example, a Morse cone fastening) or some similar connecting element capable of accurately sustaining a roller 5 intended to constitute - together with the counter roller 3 - the set of mobile cooperating elements characteristic of the processing unit 10. It is however quite evident that the solution according to the present invention can be applied also in relation to motionally cooperating elements of an altogether different type (for example, cutting and/or welding heads/jaws, applicators of various kinds, etc.).

In the case of the example here illustrated - and which is indeed no more than an example - the roller 5 is configured in the dorm of a cutting and/or welding roller provided with an embossing in relief 5a. The relief embossing 5a defines (in a manner that is known to the state of the art and, as such, does not call for any further description herein) the profile of the welding and welding/cutting zones characteristic of a hygienic or sanitary product, a sanitary towel of the type provided with wings being a case in point.

In order to enable it to act as a cutting and or welding element, the roller 5 (usually realized, just like the counter roller 3 and the greater part of the other components of the unit 10) of some metallic material, such a steel for example) carries associated with it and mounted in the in the part 4b of the shaft 4 (see Figure 2) a heating resistor 6 and a thermostatic probe 7 for controlling the operating temperature of the processing unit.

More generally, the element here exemplified by the roller 5 can consist of any operating element (be it for cutting, welding, engraving, calandering, dragging, compressing, ect.) that has appropriate temperature control elements associated with it. These latter can be either heating elements (like the resistor 6) or cooling elements and can be operated either electrically via appropriate cables or by means of various fluids passing through appropriate tubes.

The solution according to the invention proves to be particularly advantageous from this point of view, because the aforesaid temperature control organs can be associated with the part 4b of the shaft 4. In this way, indeed, neither the temperature control organs nor the cables and/or tube associated with them are in any way involved in the operation of replacing the relative element here represented by the roller 5.

This also brings out the advantage in arranging matters in such a way that the part 4b of the shaft should have a sufficiently large heat exchange surface in contact with the element 5 that it sustains. In the specific embodiment example here illustrated, this particular result is obtained, among others, by allowing the part 4b to extend inside the roller 5 for a very substantial part of the overall length of the latter.

The reference number 8 indicates a plate placed in a fixed position with respect to the framework of the unit 1. The plate 8 sustains, mounted on its underside, a pushing unit that substantially consists of a hydraulic jack 9 acting in the vertical direction. The jack 9 is fixed by means of its body frame to the plate 8, while its piston stem passes through a central opening of the plate 8 and carries a pivoting joint 9a at its upper end. At its four corners, the plate 8 sustains the straight bars or peripheral uprights that act as guide for the framework 2a that supports the counter roller 3. The latter is thus pushed upwards by the pivoting joint 9a. This arrangement is clearly such as to render the suspension of the counter roller 3 a floating one, thus enabling the counter roller to adapt to possible flexures of the cantilever structure defined by the distal part 4b of the shaft 4 and the roller 5 that is carried on it.

For clarity of explanation it should here be noted that the elements of the structures 1 and 2 that are here not explicitly mentioned or described (for example, the unit or the units for propelling the shaft 4 and/or the roller 3, the various speed controls, any such accessory elements as a blade or rake R associated with the counter roller 3, etc.) are all realized in accordance with criteria that are nowadays adopted for the realization of similar units in keeping with the state of the art. These criteria will not therefore be here discussed in any detail, not least because they are altogether irrelevant for the purposes of understanding the invention.

An important characteristic of the solution according to the invention is represented - in the embodiment here illustrated - by the modalities adopted for mounting the element here represented by the roller 5. In the illustrated embodiment, in fact, the roller 5 constitutes an active element that is intended to be changed very quickly whenever the need arises of making a rapid change of the characteristics of the product to be fabricated and/or the processing to be realized and/or simply replacing the part due to the wear and tear it has suffered in use.

In practice, the distal part 4b of the shaft 4 constitutes a so-called rapid fastening or connecting device onto which the roller 5 can be fitted with great accuracy by simply sliding it onto the part 4b, exploiting to this end the presence in the roller 5 of an axial cavity 5c having the characteristic of having exactly the same shape as the part 4b. In the specific case here illustrated, the cavity 5c therefore has a conic profile that is exactly complementary to that of the part 4b, which acts as the fastening device. Once it has been slipped onto the part 4b, the roller 5 may be fixed in position by means of fixing devices that on the whole are known. To this end, for example, use may be made of a threaded tang or key screwed into the free end of the part 4b and intended to block the roller in position via a nut 12 with the possible interposition of an annular flange 13. The latter, moreover, may also be used to maintain the roller 5 in a selectively predetermined angular position in keeping with specific application needs (for example, ensuring correct "phasing" of the unit 10 with respect to the article flow that has to be processed). In the embodiment here illustrated - and be it clear that it is, indeed, no more than such - this possibility has been represented by showing the flange 13 complete with holes 13a to permit the passage of fixing screws intended to engage the holes 5b formed on the end of the roller 5 intended to be turned towards the outside of the machine (that is to say, on the end opposite to the structure 1. The correct angular positioning of the roller 5 may thus be assured as a function of the correct angular positioning of the flange 13 with respect to the part 4b and this, in its turn, can be obtained by any known means (for example, keying, coupling of prismatic parts, etc.).

The actual operation or functioning of the unit 10 is realized in accordance with known means, that is to say, with a strip or web W of articles that usually moves forward at a substantial speed (for example, 120-450 m/min) in the space or gap between the two rollers 3 and 5. These "motionally cooperating" elements jointly define a roller/counter-roller pair positioned with the axes X3 and X4 parallel to each other, but not necessarily above each other. In fact, the forward movement of the web W may be realized in any direction whatsoever, and therefore also in a direction that is inclined either upwards or downwards and even in a completely vertical direction.

The cantilevered mounting of the roller 5 (the same solution could be adopted also for the counter-roller 3 whenever this proves necessary or useful) ensures that the replacement operation can be performed by simply bringing the unit 10 to a standstill, slackening/removing the fixing means (here represented by the nut 12, the flange 13 and the elements associated therewith) and then simply sliding the roller 5 off the distal part 4b of the shaft 4. The roller that is to be mounted as a replacement can then be placed in position by slipping it onto the part 4b. The presence of the conic cavity 5c within the roller realizes a shape adaptation that ensures perfect centering of the roller 5 with respect to the axis X4. The operation may be completed in a very brief space of time, possibly intervening in the form of slight axial taps applied to the roller 5 in the final part of its forward movement onto the part 4b. The roller 5 can then be blocked in its desired position, repositioning the fixing elements (flange 13, nut 12, etc.). The unit 10 can thereafter be restarted.

The duration of the time interval during which the unit 10 is at a standstill is therefore very short indeed, not least because the configuration of the provided fastening device ensures in an almost automatic manner that the roller 5 will be correctly positioned on conclusion of the replacement operation. It will also be appreciated that the replacement operation does not in any way involve the temperature control organs (the resistor 6, for example), and the same applies as regards the cables or tubes normally associated with them.

As has already been pointed out, the floating mounting of the counter-roller 3 provides the advantage that it permits the said roller to adjust automatically and instantaneously to any flexures of the shaft 4 and, more particularly, its distal part 4b on which the roller 5 is mounted.

Preferably, the said roller 5 is provided with at least marginally projecting flanges 14, possibly in positions corresponding to its two ends, these flanges being intended to engage with the surface of the counter-roller 3, thus ensuring that the two rollers 3 and 5 will remain separated by an interval of constant height during their motion (the interval in question being the one through which the web W advances), so that it can be accurately adapted to the particular needs of the ongoing processing operation.

Without prejudice to the principle of the invention, of course, the realization details and the embodiment forms can be varied very extensively with respect to what has here been described and illustrated without in any way going beyond the ambit of the present invention as defined on the bases of the claims attached hereto.

## Claims

1. A processing unit comprising two motionally cooperating elements (3, 5) that jointly define a zone for the advancing movement of a flow (W) of articles to be processed, characterized in that at least one (5) of the said elements has associated with it a respective supporting shaft (4) that presents a projecting distal part (49) and is mounted on the distal part (4b) of the said supporting shaft, so that the said at least one element (5) is capable of being mounted on the unit (10) and removed from the unit (10) by, respectively, mounting it on and removing it from the distal part (4b) of the said supporting shaft(4).

2. A unit in accordance with Claim 1, characterized in that the said distal part (4b) of the said supporting shaft (4) and at least one complementary part (5c) of the said at least one element (5) are configured in such a manner as to realize a rapid fastening device.

3. A unit in accordance with either Claim 1 or Claim 2, characterized in that the said distal part (4b) has a substantially tapered shape.

4. A unit in accordance with Claim 3, characterized in that the said distal part is configured as a cone fastening.

5. A unit in accordance with any one of the preceding claims, characterized in that the other (3) of the said motionally cooperating elements has associated with it means of support (2, 8, 9) of the floating type, so that the said other cooperating element (3) is capable of adapting itself to any displacements of the said at least one (5) element.

6. A unit in accordance with Claim 5, characterized in that the said other cooperating element (3) is mounted on a framework (2a) that is supported by a swivelling supporting element.

7. A unit in accordance with Claim 6, characterized in that the supporting element (9) is a pushing element capable of applying to the said other cooperating element (3) an adaptation thrust in contact with the said at least one cooperating element.

8. A unit in accordance with any one of the preceding claims, characterized in that the said motionally cooperating elements are substantially configured as a roller (5)/counter-roller (3) pair.

9. A unit in accordance with Claim 8, characterized in that at least one (5) of the said motionally cooperating elements (3, 5) is provided with cooperation formations (14) that remain in revolving relations with the other complementary element.

10. A unit in accordance with Claim 9, characterized in that the said formations are realized as projecting annular flanges (14).

11. A unit in accordance with anyone of the preceding claims, characterized in that with the said at least one element (5) there are associated temperature control means (6, 7) and that the said temperature control means (6, 7) act on the said distal part (4b) of the said supporting shaft (4).

12. A unit in accordance with Claim 11, characterized in that the said at least one element (5) presents a substantial heat exchange surface (5a) with the said distal part (4) of the said supporting shaft (4).

13. A unit in accordance with any one of the preceding claims, characterized in that the said unit is a unit for processing hygienic and sanitary products (W).
